# EUROPEAN PATENT SPECIFICATION

(11) **EP 1 147 133 B1**
(45) Date of publication and mention of the grant of the patent: **06.08.2008**
(21) Application number: 99968197.6
(22) Date of filing: 30.12.1999
(51) Int. Cl.: C07K 14/635, A61K 38/29, C12N 15/00

(54) **Peptides consisting of two PTH functional domains fused by a linker molecule, and derivatives thereof**
Peptide, bestehend aus zwei funktionellen PTH-Domänen, die durch ein Linkermolekül verknüpft sind, und Derivate davon
Peptides comprenant une fusion de deux domaines fonctionnels de la PTH par une molecule linker, et leurs dérivés

(30) Priority: 31.12.1998 US 114577 P
(43) Date of publication of application: 24.10.2001
(73) Proprietor: THE GENERAL HOSPITAL CORPORATION, Boston, MA 02114 (US)
(72) Inventor: GARDELLA, Thomas, J, Cambridge , MA 02139 (US); KRONENBERG, Henry M., Belmont, MA 02478 (US); POTTS,John T. Jr., Newton,MA 02165 (US); JÜPPNER, Harald, Boston, MA 02116 (US)
(74) Representative: Chapman, Paul William
(86) International application number: PCT/US1999/031108
(87) International publication number: WO 2000/039278

(56) References cited:
- EP-A- 0 561 412
- WO-A-95/11988
- GB-A- 2 269 176
- US-A- 4 086 196
- US-A- 5 556 940
- GARDELLA T J ET AL: "Parathyroid hormone (PTH)-PTH-related peptide hybrid peptides reveal functional interactions between the 1-14 and 15-34 domains of the ligand" JOURNAL OF BIOLOGICAL CHEMISTRY, vol. 270, no. 12, 24 March 1995 (1995-03-24), pages 6584-6588, XP002046996 ISSN: 0021-9258
- TAKASU HISASHI ET AL: "Type-1 parathyroid hormone (PTH)/PTH-related peptide (PTHrP) receptors activate phospholipase C in response to carboxyl-truncated analogs of PTH(1-34)." ENDOCRINOLOGY, vol. 139, no. 10, October 1998 (1998-10), pages 4293-4299, XP002205690 ISSN: 0013-7227
- PELLEGRINI MARIA ET AL: "Addressing the tertiary structure of human parathyroid hormone-(1-34)." JOURNAL OF BIOLOGICAL CHEMISTRY, vol. 273, no. 17, 24 April 1998 (1998-04-24), pages 10420-10427, XP002205691 ISSN: 0021-9258

## Description

### Background of the Invention

### Field of the Invention

The present invention is related to the fields of molecular biology, developmental biology, physiology, neurobiology, endocrinology and medicine.

### Description of Related Art

Parathyroid hormone (PTH) is a major regulator of calcium homeostasis whose principal target cells occur in bone and kidney. Regulation of calcium concentration is necessary for the normal function ofthe gastrointestinal, skeletal, neurologic, neuromuscular, and cardiovascular systems. PTH synthesis and release are controlled principally by the serum calcium level; a low level stimulates and a high level suppresses both hormone synthesis and release. PTH, in turn, maintains the serum calcium level by directly or indirectly promoting calcium entry into the blood at three sites of calcium exchange: gut, bone, and kidney. PTH contributes to net gastrointestinal absorption of calcium by favoring the renal synthesis of the active form of vitamin D. PTH promotes calcium resorption from bone indirectly by stimulating differentiation of the bone-resorbing cells, osteoclasts. It also mediates at least three main effects on the kidney: stimulation of tubular calcium reabsorption, enhancement of phosphate clearance, and promotion of an increase in the enzyme that completes synthesis of the active form of vitamin D. PTH exerts these effects primarily through receptor-mediated activation of adenylate cyclase and phospholipase C.

Disruption of calcium homeostasis may produce many clinical disorders (*e*.*g*., severe bone disease, anemia, renal impairment, ulcers, myopathy, and neuropathy) and usually results from conditions that produce an alteration in the level of parathyroid hormone. Hypercalcemia is a condition that is characterized by an elevation in the serum calcium level. It is often associated with primary hyperparathyroidism in which an excess of PTH production occurs as a result of a lesion (*e*.*g*., adenoma, hyperplasia, or carcinoma) of the parathyroid glands. Another type of hypercalcemia, humoral hypercalcemia of malignancy (HHM) is the most common paraneoplastic syndrome. It appears to result in most instances from the production by tumors (*e*.*g*., squamous, renal, ovarian, or bladder carcinomas) of a class of protein hormone which shares amino acid homology with PTH. These PTH-related proteins (PTHrP) appear to mimic certain of the renal and skeletal actions of PTH and are believed to interact with the PTH receptor in these tissues. PTHrP is normally found at low levels in many tissues, including keratinocytes, brain, pituitary, parathyroid, adrenal cortex, medulla, fetal liver, osteoblast-like cells, and lactating mammary tissues. In many HHM malignancies, PTHrP is found in the circulatory system at high levels, thereby producing the elevated calcium levels associated with HHM.

The pharmacological profiles of PTH and PTHrP are nearly identical in most *in vitro* assay systems, and elevated blood levels of PTH (*i*.*e*., primary hyperparathyroidism) or PTHrP (*i*.*e*., HHM) have comparable effects on mineral ion homeostasis (Broadus, A.E. & Stewart, A.F., "Parathyroid hormone-related protein: Structure, processing and physiological actions," in Basic and Clinical Concepts, Bilzikian, J.P. et al., eds., Raven Press, New York (1994), pp. 259-294; Kronenberg, H.M. et al., "Parathyroid hormone: Biosynthesis, secretion, chemistry and action," in Handbook of Experimental Pharmacology, Mundy, G.R. & Martin, T.J., eds., Springer-Verlag, Heidelberg (1993), pp. 185-201). The similarities in the biological activities of the two ligands can be explained by their interaction with a common receptor, the PTH/PTHrP receptor, which is expressed abundantly in bone and kidney (Urena, P. et al., Endocrinology 134:451-456 (1994)).

Native human parathyroid hormone is an unmodified polypeptide of 84 amino acids. It is secreted from the parathyroid glands in response to low blood calcium levels and acts on osteoblast (bone-building cells) in bone, and on tubular epithelial cells of kidney. The hormone interacts with a cell surface receptor molecule, called the PTH-1 receptor or PTH/PTHrP receptor, which is expressed by both osteoblast and renal tubular cells. PTHrP, the major cause of the humoral hypercalcemia of malignancy, also has normal functions that include roles in development. PTHrP has 141 amino acids, though variants also occur that result from alternative gene splicing mechanisms. PTHrP plays a key role in the formation of the skeleton through a process that also involves binding to the PTH-1 receptor (Karaplis, A.C., et al., Genes and Dev. 8:277-289 (1994) and Lanske, B., et al., Science 273:663-666 (1996)).

The PTH-1 receptor is homologous in primary structure to a number of other receptors that bind peptide hormones, such as secretin (Ishihara, T. et al., EMBO J. 10:1635-1641 (1991)), calcitonin (Lin, H.Y. et al., Science 254:1022-1024 (1991)) and glucagon (Jelinek, L.J. et al., Science 259:1614-1616 (1993)); together these receptors form a distinct family called receptor family B (Kolakowski, L.F., Receptors and Channels 2:1-7 (1994)). Within this family, the PTH-1 receptor is unique, in that it binds two peptide ligands and thereby regulates two separate biological processes. A recently identified PTH receptor subtype, called the PTH-2 receptor, binds PTH but not PTHrP (Usdin, T., et al., J. Biol. Chem. 270:15455-15458 (1995)). This observation implied that structural differences in the PTH and PTHrP ligands determined selectivity for interaction with the PTH-2 receptor. The PTH-2 receptor has been detected by RNA methods in the brain, pancreas and vasculature, however, its biological function has not been determined (Usdin, T., et al., J. Biol. Chem. 270:15455-15458 (1995)). It is hypothesized that the family B receptors use a common molecular mechanism to engage their own cognate peptide hormone (Bergwitz, C., et al., J. Biol. Chem. 271:26469-26472 (1996)).

The binding of either radiolabeled PTH(1-34) or PTHrP(1-36) to the PTH-1 receptor is competitively inhibited by either unlabeled ligand (Jüppner, H. et al., J. Biol. Chem. 263:8557-8560 (1988); Nissenson, R.A. et al., J. Biol. Chem. 263:12866-12871 (1988)). Thus, the recognition sites for the two ligands in the PTH-1 receptor probably overlap. In both PTH and PTHrP, the 15-34 region contains the principal determinants for binding to the PTH-1 receptor. Although these regions show only minimal sequence homology (only 3 amino acid identities), each 15-34 peptide can block the binding of either PTH(1-34) or PTHrP(1-34) to the PTH-1 receptor (Nussbaum, S.R. et al., J. Biol. Chem. 255:10183-10187 (1980); Caulfield, M.P. et al., Endocrinology 127:83-87 (1990); Abou-Samra, A.-B. et al., Endocrinology 125:2215-2217 (1989)). Further, the amino terminal portion of each ligand is required for bioactivity, and these probably interact with the PTH-1 receptor in similar ways, since 8 of 13 of these residues are identical in PTH and PTHrP.

Both PTH and PTHrP bind to the PTH-1 receptor with affinity in the nM range; the ligand-occupied receptor transmits a "signal" across the cell membrane to intracellular effector enzymes through a mechanism that involves intermediary heterotrimeric GTP-binding proteins (G proteins). The primary intracellular effector enzyme activated by the PTH-1 receptor in response to PTH or PTHrP is adenylyl cyclase (AC). Thus, PTH induces a robust increase in the "second messenger" molecule, cyclic adenosine monophosphate (cAMP) which goes on to regulate the poorly characterized "downstream" cellular processes involved in bone-remodeling (both bone formation and bone resorption processes). In certain cell-based assay systems, PTH can stimulate effector enzymes other than AC, including phospholipase C (PLC), which results in production of inositol triphosphate (IP₃), diacylglycerol (DAG) and intracellular calcium (iCa²⁺). The roles of these non-cAMP second messenger molecules in bone metabolism are presently unknown.

Osteoporosis is a potentially crippling skeletal disease observed in a substantial portion of the senior adult population, in pregnant women and even in juveniles. The disease is marked by diminished bone mass, decreased bone mineral density (BMD), decreased bone strength and an increased risk of bone fracture. At present, there is no effective cure for osteoporosis, though estrogen, calcitonin and the bisphosphonates, etidronate and alendronate are used to treat the disease with varying levels of success through their action to decrease bone resorption. Since parathyroid hormone regulates blood calcium and the phosphate levels, and has potent anabolic (bone-forming) effects on the skeleton, in animals (Shen, V., et al., Calcif. Tissue Int. 50:214-220 (1992); Whitefild, J.F., et al., Calcif. Tissue Int. 56:227-231 (1995) and Whitfield, J.F., et al., Calcif. Tissue Int. 60:26-29 (1997)) and humans (Slovik, D.M., et al., J. Bone Miner. Res. 1:377-381 (1986); Dempster, D.W., et al., Endocr. Rev. 14:690-709 (1993) and Dempster, D.W., et al., Endocr. Rev. 15:261 (1994)) when administered intermittently, PTH, or PTH derivatives, are prime candidates for new and effective therapies for osteoporosis.

Truncated PTH derivatives such as PTH(1-34) and PTH(1-31) are active in most assay systems and promote bone-formation (Whitefild, J.F., et al., Calcif. Tissue Int. 56:227-231 (1995); Whitfield, J.F., et al., Calcif. Tissue Int. 60:26-29 (1997); Slovik, D.M., et al., J. Bone Miner. Res. 1:377-381 (1986); Tregear, G.W., et al., Endocrinology 93:1349-1353 (1973); Rixon, R.H., et al., J. Bone Miner. Res. 9:1179-1189 (1994); Whitfield, J.F. and Morley, P., Trends Pharmacol. Sci. 16:372-386 (1995) and Whitfield, J.F., et al., Calcif. Tissue Int. 58:81-87 (1996)). But these peptides are still too large for efficient non-parenteral delivery and low cost. The discovery of an even smaller "minimized" version of PTH or PTHrP would be an important advance in the effort to develop new treatments for osteoporosis.

PTH and PTHrP derivatives that have amino acid substitutions or deletions in the 1-14 region usually exhibit diminished activity (Tregear, G.W., et al., Endocrinology 93:1349-1353 (1973); Goltzman, D., et al., J. Biol. Chem. 250:3199-3203 (1975); Horiuchi, N., et al., Science 220:1053-1055 (1983) and Gardella, T.J., et al., J. Biol. Chem. 266:13141-13146 (1991))

Several short NH₂-terminal PTH or PTHrP peptides have been investigated previously, but no activity was detected. For example, bPTH(1-12) was inactive in adenylyl cyclase assays performed in rat renal membranes (Rosenblatt, M., "Parathyroid Hormone: Chemistry and Structure-Activity Relations," in Pathobiology Annual, Ioachim, H.L., ed., Raven Press, New York (1981), pp. 53-84) and PTHrP(1-16) was inactive in AC assays performed in Chinese hamster ovary (CHO) cells expressing the cloned rat PTH-1 receptor (Azurani, A., et al., J. Biol. Chem. 271:14931-14936 (1996)). It has been known that residues in the 15-34 domain of PTH contribute importantly to receptor binding affinity, as the PTH(15-34) fragment binds weakly to the receptor, but this peptide does not activate AC (Naussbaum, S.R., et al., J. Biol. Chem. 255:10183-10187 (1980) and Gardella, T.J., et al., Endocrinology 132:2024-2030 (1993)).

### Summary of the Invention

The relatively large size of native PTH or PTHrP presents challenges to the use of these peptides as treatments for osteoporosis. In general, a protein of this size is not suitable for use as a drug, since it cannot be delivered effectively by simple methods such as nasal inhalation. Instead, injection is required, and in the case of PTH, daily, or almost daily injections would most likely be needed to achieve increases in bone formation rates. Additionally, larger peptides are technically difficult and expensive to prepare by conventional synthetic chemistry methods. Alternative methods employing recombinant DNA and cell-based expression systems are also expensive, potentially vulnerable to contamination by foreign proteins and do not circumvent the delivery problem.

Accordingly, it would be advantageous for those skilled in the art to be able to identify a small molecule analog (either peptide or non-peptide) that is based on the larger peptide and yet which still retains the desired biological activities. The activity may at first be weak relative to the intact peptide, but further optimization can lead to enhanced efficacy and potency.

The present invention relates to compound of the formula or structure S-(L)ₙ-B, wherein S is an amino terminal signaling functional domain of PTH consisting of PTH(1-9): Ala Val Ser Glu Ile Gln Leu Met His (SEQ ID NO: 1); L is a linker molecule present n times, wherein n is 5-9; B is a C-terminal binding portion of PTH(1-34) consisting of a PTH(15-31): Leu Asn Ser Met Glu Arg Val Glu Trp Leu Arg Lys Lys Leu Gln Asp Val (SEQ ID NO:2); and wherein L is glycine. The compound is preferably an isolated peptide or polypeptide. This aspect of the invention also relates to C-derivatives derived from these S-(L)n-B peptides by addition of C₁₋₁₂ alkyl groups, the addition of C₁₋₁₂ hydroxyalkyl groups, the addition of acyl groups or lactam cyclization and are hereinafter collectively referred to as "S-(L)n-B compounds of the invention and derivatives thereof".

In accordance with yet a further aspect of the invention, this invention provides the use of the polypeptide of the invention in the manufacture of a reagent for the treatment of mammalian hypercalcaemia or mammalian hypocalcaemia.

In accordance with yet a further aspect of the invention, this invention also provides the use of a polypeptide of the invention in the manufacture of a reagent for determining rates of bone reformation, bone resorption and/or bone remodelling.
The peptide may be labelled with a label selected from the group consisting of: radiolabel, fluorescent label, bioluminescent label, or chemiluminescent label. An example of a suitable radiolabel is ^{99m}Tc.

The invention is further directed to the use of the polypeptide of the invention in the manufacture of a reagent for treating mammalian hypercalcaemia or mammalian hypocalcaemia. Another aspect of the invention involves using DNA encoding said peptide in the manufacture of a medicament to treat the same condition. Preferably the condition to be treated is osteoporosis. Administration of the polypeptide may be by any methods known to those of skill in the art preferably at an effective amount of said polypeptide from about 0.01 µg/kg/day to about 1.0 µg/kg/day.

The invention is further directed to increasing intracellular cAMP in a mammalian cell having PTH-1 receptors in vitro, comprising contacting said cell with a sufficient amount of the polypeptides of the invention to increase intracellular cAMP.

### Brief Description of the Figures

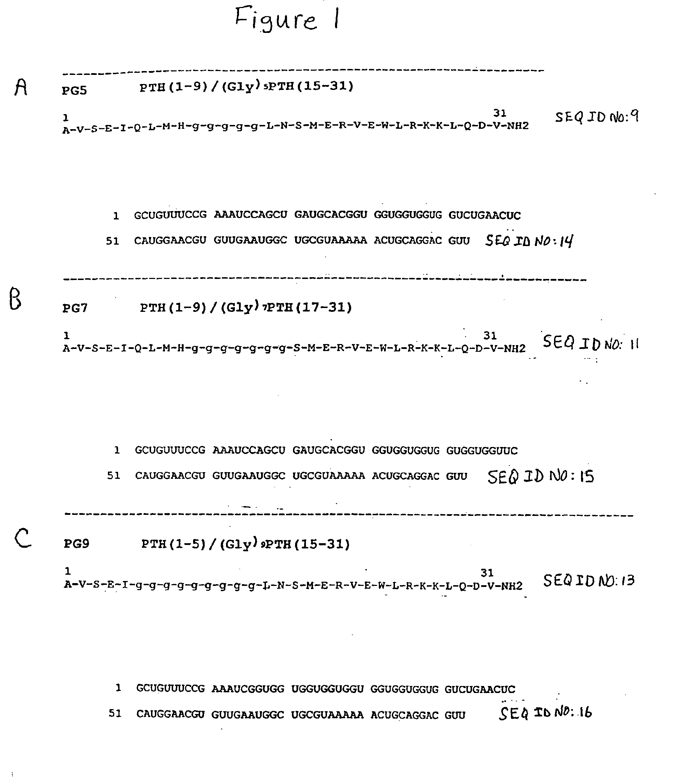

Figure 1. Presentation of PG5 (PTH (1-9)-(Gly)-₅PTH(15-31)) amino acid (SEQ ID NOS: 3 and 9) and nucleic acid (SEQ ID NO: 14) sequence; PG7 (PTH (1-9)-(Gly)₇-PTH(17-31)) amino acid (SEQ ID NOS:6 and 11) and nucleic acid (SEQ ID NO:15) sequence; and PG9 (PTH (1-5)-(Gly)₉-PTH(15-31)) amino acid (SEQ ID NOS: 5 and 13) and nucleic acid (SEQ ID NO:16) sequence.

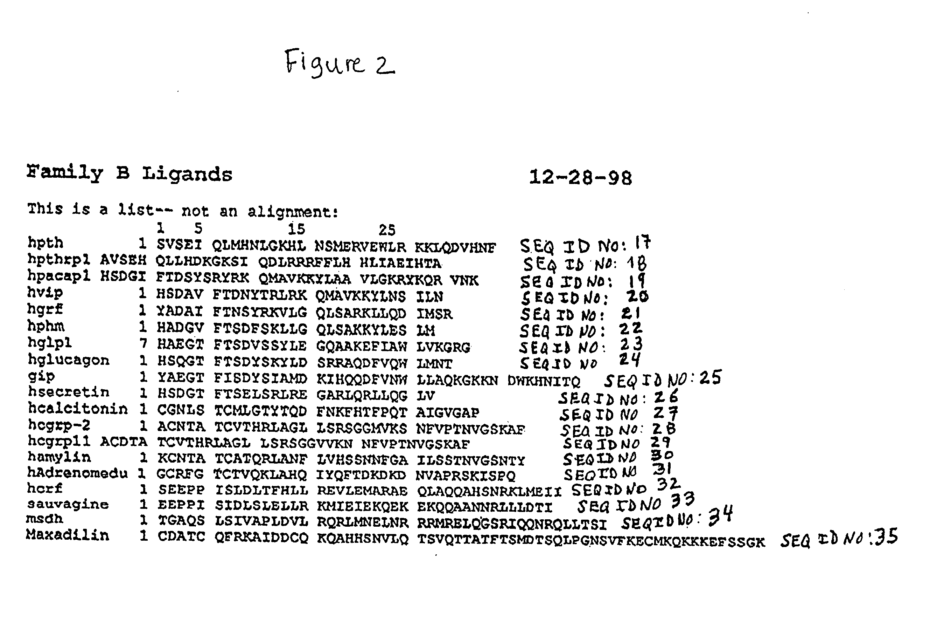

Figure 2. Presentation of the G protein receptor coupled B family ligand amino-terminal sequences.

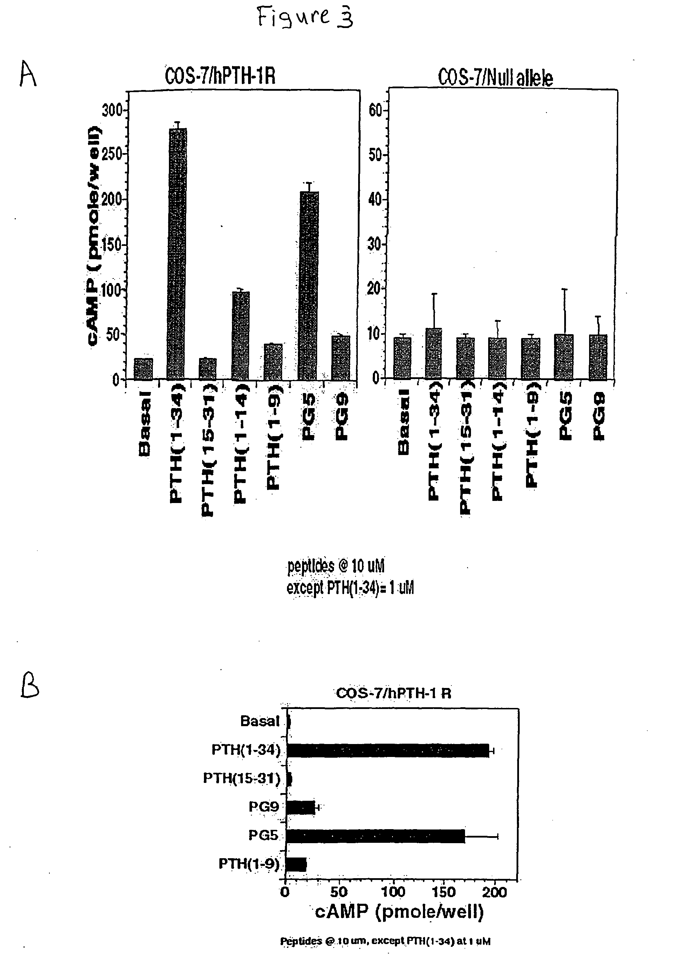

Figure 3. Measurement of the total accumulation of cyclic AMP (cAMP) in COS cells in response to exposure to the indicated PTH peptides. A) Comparison of the accumulation of total cAMP in COS cells expressing human PTH-1 receptor and cells expressing a translation-stop, null PTH-1 receptor. B) Presentation of a second experiment demonstrating the effect of PG5 and PG9 on the accumulation of cAMP in COS cells expressing the human PTH-1 receptor.

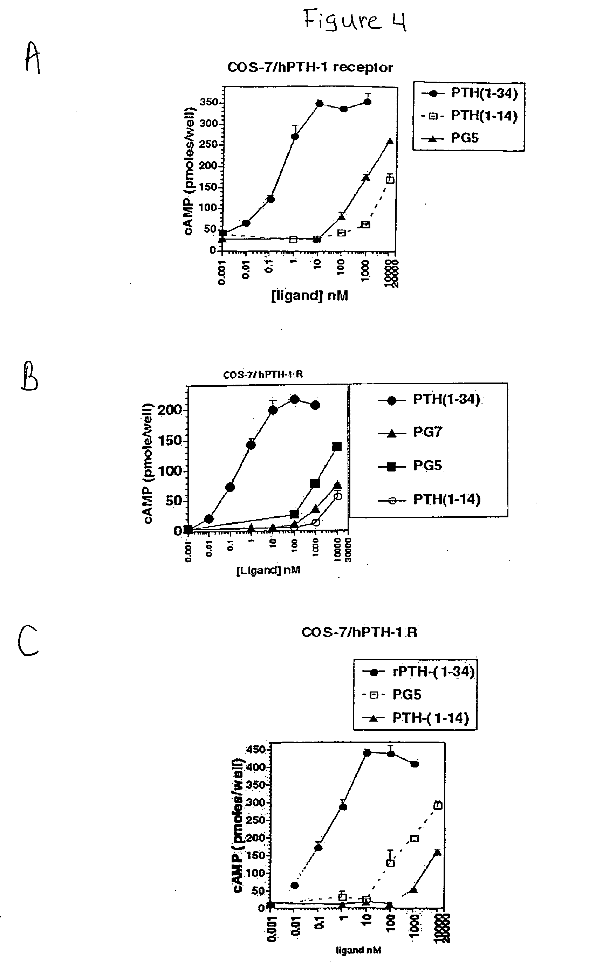

Figure 4. cAMP dose response curves of short amino-terminal PTH analogs in COS cells transfected with the human PTH-1 receptor (COS7/hPTH-1 cells). COS cells in 24 plates were treated with the indicated peptides for 60 mins at 21°C, and then intracellular cAMP levels were measured. All PTH peptides shown are based on the rat PTH sequence and are carboxy-terminally amidated. 4A, 4B and 4C represent separate experiments.

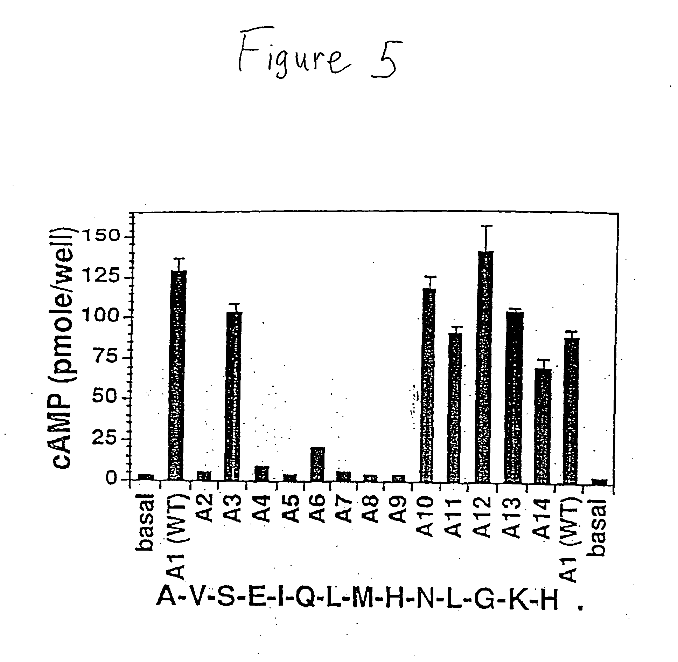

Figure 5. Alanine-scan of PTH(1-14). Shown are the bioactivities of 14 different PTH(1-14) derivatives, each having a different amino acid of the native sequence (shown at bottom of figure) replaced by alanine. Peptides were chemically synthesized, purified and tested for ability to stimulate cAMP formation in COS-7 cells expressing the cloned human PTH-1 receptor. Peptides were tested in duplicate (± s.e.m.) at a dose of 67 µM. As a control, untreated cells, indicated by basal, were measured. The PTH(1-14) containing alanine at position 1 was used as the wild-type reference. Cells were stimulated for 30 minutes at 21°C. This figure provides information relevant to bioactivities of amino acid residues in the PTH(1-9) peptide used in the invention.

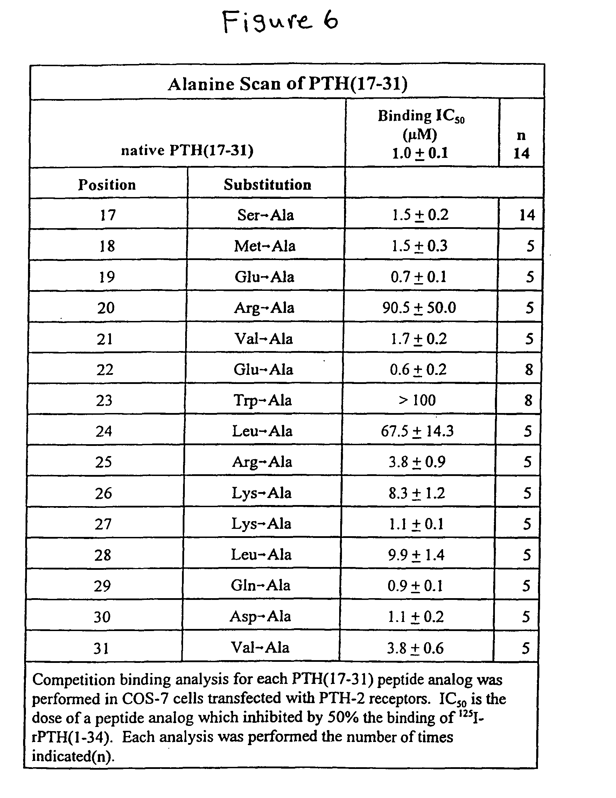

Figure 6. Alanine-scan of PTH(17-31). Shown are the results of competition binding analysis, expressed in IC₅₀ values of 15 different PTH(17-31) derivatives, each having a different amino acid of the native sequence (shown at bottom of figure) replaced by alanine. Peptides were chemically synthesized, purified and tested for ability to inhibit PTH(1-34) binding to COS-7 cells expressing the cloned human PTH-2 receptor. IC₅₀ is the dose of a peptide required to inhibit 50% of ¹²⁵I PTH(1-34) binding. This figure provides information relevant to bioactivities of amino acid residues in the PTH(17-31) peptide used in the invention.

### Detailed Description of the Preferred Embodiments

In order to provide a clearer understanding ofthe specification and claims, the following definitions are provided.

### 1. Definitions

In the description that follows, a number of terms used in recombinant DNA technology and peptide synthesis are utilized extensively. In order to provide a clear and consistent understanding of the specification and claims, including the scope to be given such terms, the following definitions are provided.

***Cloning vector:*** A plasmid or phage DNA or other DNA sequence which is able to replicate autonomously in a host cell, and which is characterized by one or a small number of restriction endonuclease recognition sites at which such DNA sequences may be cut in a determinable fashion without loss of an essential biological function of the vector, and into which a DNA fragment may be spliced in order to bring about its replication and cloning. The cloning vector may further contain a marker suitable for use in the identification of cells transformed with the cloning vector. Markers, for example, provide tetracycline resistance or ampicillin resistance.

***Expression vector:*** A vector similar to a cloning vector but which is capable of enhancing the expression of a gene which has been cloned into it, after transformation into a host. The cloned gene is usually placed under the control of (*i*.*e*., operably linked to) certain control sequences such as promoter sequences. Promoter sequences may be either constitutive or inducible.

***Recombinant Host:*** A recombinant host may be any prokaryotic or eukaryotic host cell which contains the desired cloned genes on an expression vector or cloning vector. This term is also meant to include those prokaryotic or eukaryotic cells that have been genetically engineered to contain the desired gene(s) in the chromosome or genome of that organism. For examples of such hosts, *see* Sambrook et al., Molecular Cloning: A Laboratory Manual, Second Edition, Cold Spring Harbor Laboratory, Cold Spring Harbor, New York (1989). Preferred recombinant hosts are eukaryotic cells transformed with the DNA construct of the invention. More specifically, mammalian cells are preferred.

***Promoter:*** A DNA sequence generally described as the 5' region of a gene, located proximal to the start codon. The transcription of an adjacent gene(s) is initiated at the promoter region. If a promoter is an inducible promoter, then the rate of transcription increases in response to an inducing agent. In contrast, the rate of transcription is not regulated by an inducing agent if the promoter is a constitutive promoter. Examples of promoters include the CMV promoter (InVitrogen, San Diego, CA), the SV40, MMTV, and hMT11a promoters (U.S. Pat. No. 5,457,034), the HSV-1 4/5 promoter (U.S. Pat. No. 5,501,979), and the early intermediate HCMV promoter (WO92/17581). Also, tissue-specific enhancer elements may be employed. Additionally, such promoters may include tissue and cell-specific promoters of an organsim.

***Polynucleotide:*** This term generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety ofmodifications have been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

***Polypeptide:*** This term refers to any peptide or protein comprising two or more amino acids joined to each other by peptide bonds or modified peptide bonds, *i*.*e*., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as post-translational processing, or by chemical modification techniques which are well known in the art. Such modifications are well described in basic texts and in more detailed monographs, as well as in the research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications.

Polypeptides may be branched and they may be cyclic, with or without branching. Cyclic, branched and branched cyclic polypeptides may result from post-translation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent crosslinks, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. *See*, for instance, Proteins-Structure and Molecular Properties, 2nd Ed., T. E. Creighton, W. H. Freeman and Company, New York, 1993 and Wold, F., Posttranslational Protein Modifications: Perspectives and Prospects, pgs. 1-12 in Posttranslational Covalent Modification of Proteins, B. C. Johnson, Ed., Academic Press, New York, 1983; Seifter et al., "Analysis for protein modifications and nonprotein cofactors", Methods in Enzymol. 182:626-646 (1990) and Rattan et al., "Protein Synthesis: Posttranslational Modifications and Aging", Ann NY Acad Sci 663:48-62 (1992). The polypeptides of the invention have a free amino group at the N-terminus and a carboxy-amid at the C-terminus.

***Homologous*/*Nonhomologous:*** Two nucleic acid molecules are considered to be "homologous" if their nucleotide sequences share a similarity of greater than 40%, as determined by HASH-coding algorithms (Wilber, W.J. and Lipman, D.J., Proc. Natl. Acad. Sci. 80:726-730 (1983)). Two nucleic acid molecules are considered to be "nonhomologous" if their nucleotide sequences share a similarity of less than 40%.

***Isolated:*** A term meaning altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein. Thus, a polypeptide or polynucleotide produced and/or contained within a recombinant host cell is considered isolated for purposes of the present invention. Also intended as an "isolated polypeptide" or an "isolated polynucleotide" are polypeptides or polynucleotides that have been purified, partially or substantially, from a recombinant host cell or from a native source. For example, a recombinantly produced version of compounds of SEQ ID NO:1 and derivatives thereof can be substantially purified by the one-step method described in Smith and Johnson, Gene 67:31-40 (1988).

By "isolated" is meant that the DNA is free of the coding sequences of those genes that, in the naturally-occurring genome of the organism (if any) from which the DNA of the invention is derived, immediately flank the gene encoding the DNA of the invention. The isolated DNA may be single-stranded or double-stranded, and may be genomic DNA, cDNA, recombinant hybrid DNA, or synthetic DNA. It may be identical to a native DNA sequence encoding compounds of SEQ ID NO:1 Single-stranded DNAs are generally at least 8 nucleotides long, (preferably at least 18 nucleotides long, and more preferably at least 30 nucleotides long) ranging up to full length of the DNA molecule encoding compounds of SEQ ID NO:1 and derivatives thereof (*i*.*e*., 42 nucleotides); they preferably are detectably labeled for use as hybridization probes, and may be antisense.

Isolated or purified as it refers to preparations made from biological cells or hosts should be understood to mean any cell extract containing the indicated DNA or protein including a crude extract of the DNA or protein of interest. For example, in the case of a protein, a purified preparation can be obtained following an individual technique or a series of preparative or biochemical techniques and the DNA or protein of interest can be present at various degrees of purity in these preparations. The procedures may include for example, but are not limited to, ammonium sulfate fractionation, gel filtration, ion exchange change chromatography, affinity chromatography, density gradient centrifugation and electrophoresis.

A preparation of DNA or protein that is "pure" or "isolated" should be understood to mean a preparation free from naturally occurring materials with which such DNA or protein is normally associated in nature. "Essentially pure" should be understood to mean a "highly" purified preparation that contains at least 95% of the DNA or protein of interest.

A cell extract that contains the DNA or protein of interest should be understood to mean a homogenate preparation or cell-free preparation obtained from cells that express the protein or contain the DNA of interest. The term "cell extract" is intended to include culture media, especially spent culture media from which the cells have been removed.

While many embodiments of the claimed invention use isolated or purified polynucleotides or polypeptides, this need not always be the case.

***High Stringency:*** By "high stringency" is meant, for example, conditions such as those described for the isolation of cDNA (also see Current Protocols in Molecular Biology, John Wiley & Sons, New York (1989)). The DNA of the invention may be incorporated into a vector which may be provided as a purified preparation (e.g., a vector separated from the mixture of vectors which make up a library,) containing a DNA sequence encoding a peptide of the invention (e.g. compounds of SEQ ID NO: and derivatives thereof) and a cell or essentially homogenous population of cells (e.g., prokaryotic cells, or eukaryotic cells such as mammalian cells) which contain the vector (or the isolated DNA described above).

***Identity:*** This term refers to a measure of the identity of nucleotide sequences or amino acid sequences. In general, the sequences are aligned so that the highest order match is obtained. "Identity" *per se* has an art-recognized meaning and can be calculated using published techniques. (See, *e*.*g*.: Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). While there exist a number of methods to measure identity between two polynucleotide or polypeptide sequences, the term "identity" is well known to skilled artisans (Carillo, H. & Lipton, D., SIAM J Applied Math 48:1073 (1988)). Methods commonly employed to determine identity or similarity between two sequences include, but are not limited to, those disclosed in Guide to Huge Computers, Martin J. Bishop, ed., Academic Press, San Diego, 1994, and Carillo, H. & Lipton, D., SIAM J Applied Math 48:1073 (1988). Methods to determine identity and similarity are codified in computer programs. Preferred computer program methods to determine identity and similarity between two sequences include, but are not limited to, GCG program package (Devereux, J., et al., Nucleic Acids Research 12(i):387 (1984)), BLASTP, BLASTN, FASTA (Atschul, S.F., et al., J Molec Biol 215:403 (1990)).

Therefore, as used herein, the term "identity" represents a comparison between a test and reference polypeptide. More specifically, reference test polypeptide is defined as any polypeptide that is 85% or more identical to a reference polypeptide. As used herein, the term at least 85% identical to refers to percent identities from 85 to 99.99 relative to the reference polypeptides. Identity at a level of 85% or more is indicative of the fact that, assuming for exemplification purposes a test and reference polynucleotide length of 100 amino acids, that no more than 15% (i.e., 15 out of 100) amino acids in the test polypeptides differ from that of the reference polypeptides. Such differences may be represented as point mutations randomly distributed over the entire length of the amino acid sequence of the invention or they may be clustered in one or more locations of varying length up to the maximum allowable 2/14 amino acid difference (approximately 85% identity). Differences are defined as amino acid substitutions, or deletions.

***Fragment:*** A "fragment" of a molecule such as a compound of SEQ ID NO: 1 or derivative thereof is meant to refer to any polypeptide subset of these molecules.

***Functional Derivative:*** The term "derivatives" is intended to include "variants," the "derivatives," or "chemical derivatives" of the molecule. A "variant" of a molecule such as a compound of SEQ ID NO: I or derivative thereof is meant to refer to a molecule substantially similar to either the entire molecule, or a fragment thereof An "analog" of a molecule such as a compound of SEQ ID NO: 1 or derivative thereof is meant to refer to a non-natural molecule substantially similar to either the SEQ ID NO: 1 molecules or fragments thereof.

A molecule is said to be "substantially similar" to another molecule if the sequence of amino acids in both molecules is substantially the same, and if both molecules possess a similar biological activity. Thus, provided that two molecules possess a similar activity, they are considered variants, derivatives, or analogs as that term is used herein even if one of the molecules contains additional amino acid residues not found in the other, or if the sequence of amino acid residues is not identical.

As used herein, a molecule is said to be a "chemical derivative" of another molecule when it contains additional chemical moieties not normally a part of the molecule. Such moieties may improve the molecule's solubility, absorption, biological half-life, etc. The moieties may alternatively decrease the toxicity ofthe molecule, eliminate or attenuate any undesirable side effect of the molecule, etc. Examples of moieties capable of mediating such effects are disclosed in Remington's Pharmaceutical Sciences (1980) and will be apparent to those of ordinary skill in the art.

***Biological Activity of the Protein:*** This expression refers to the metabolic or physiologic function of compounds, for example, SEQ ID NO: 1 or derivatives thereof including similar activities or improved activities or those activities with decreased undesirable side-effects. Also included are antigenic and immunogenic activities of said compounds of, for example, SEQ ID NO: 1 or derivatives thereof.

***Fusion protein:*** By the term "fusion protein" is intended a fused protein comprising compounds of for example, SEQ ID NO: 1 or derivatives thereof, either with or without a "selective cleavage site" linked at its N-terminus, which is in turn linked to an additional amino acid leader polypeptide sequence.

***Selective cleavage site:*** The term "selective cleavage site" refers to an amino acid residue or residues which can be selectively cleaved with either chemicals or enzymes in a predictable manner. A selective enzyme cleavage site is an amino acid or a peptide sequence which is recognized and hydrolyzed by a proteolytic enzyme. Examples of such sites include, without limitation, trypsin or chymotrypsin cleavage sites.

***Leader Sequence:*** By the term "leader sequence" is intended a polynucleotide sequence linked to for example, DNA encoding compounds of SEQ ID NO: 1, and expressed in host cells as a fusion protein fused to the selective cleavage site and compounds of SEQ ID NO: 1. The term "leader polypeptide" describes the expressed form of the "leader sequence" as obtained in the fusion protein.

The fusion protein, which is often insoluble and found in inclusion bodies when it is overexpressed, is purified from other bacterial protein by methods well known in the art. The insoluble fusion protein is centrifuged and washed after cell lysis, and resolubilized with guanidine-HCI. It can remain soluble after removal of the denaturant by dialysis. (For purification of refractile proteins, see Jones, U.S. Pat. No. 4,512922; Olson, U.S. Pat. No. 4,518,526; and Builder et al., U.S. Pat. Nos. 4,511,502 and 4,620,948).

The recombinantly produced compounds of, for example, SEQ ID NO: 1 or derivatives thereof can be purified to be substantially free of natural contaminants from the solubilized fusion protein through the use of any of a variety of methodologies. As used herein, a compound is said to be "substantially free of natural contaminants" if it has been substantially purified from materials with which it is found following expression in bacterial or eukaryotic host cells. Compounds of SEQ ID NO: I or derivatives thereof may be purified through application of standard chromatographic separation technology.

Alternatively, the peptide may be purified using immuno-affinity chromatography (Rotman, A. et al., Biochim. Biophys. Acta 641:114-121 (1981); Sairam, M. R. J,. Chromatog 215:143-152 (1981); Nielsen, L. S. et al., Biochemistry 21:6410-6415 (1982); Vockley, J. et al., Biochem. J. 217:535-542 (1984*)*; Paucha, E. et al., J. Virol. 51:670-681 (1984*)*; *and* Chong, P. et al., J. Virol. Meth. 10:261-268 (1985*)).*

After partial or substantial purification, the fusion protein is treated enzymatically with the enzyme corresponding to the cleavage site. Alternatively, the fusion protein in its more impure state, even in refractile form, can be treated with the enzyme. If needed, the resulting mature compounds of SEQ ID NO: 1 or derivatives thereof, can be further purified. Conditions for enzymatic treatment are known to those of skill in the art.

***Gene Therapy*:** A means of therapy directed to altering the normal pattern of gene expression of an organism. Generally, a recombinant polynucleotide is introduced into cells or tissues of the organism to effect a change in gene expression.

***Host Animal:*** Non-human transgenic animals, all of whose germ and somatic cells contain the DNA construct of the invention. Such transgenic animals are in general vertebrates. Preferred host animals are mammals such as non-human primates, mice, sheep, pigs, cattle, goats, guinea pigs, rodents, e.g. rats, and the like. The term Host Animal also includes non-human animals in all stages of development, including embryonic and fetal stages.

***Osteoporosis:*** Osteoporosis is a potentially crippling skeletal disease observed in a substantial portion of the senior adult population, in pregnant women and even in juveniles. The term osteoporosis refers to a heterogeneous group of disorders. Clinically, osteoporosis is separated into type I and type II. Type I osteoporosis occurs predominantly in middle aged women and is associated with estrogen loss at menopause, while osteoporosis type II is associated with advancing age. Patients with osteoporosis would benefit from new therapies designed to promote fracture repair, or from therapies designed to prevent or lessen the fractures associated with the disease.

### 2. Novel PTH Functional Domain Conjugate Peptides of the Formula S-(L)ₙ-B

In a first embodiment, the invention provides for novel PTH functional domain conjugate peptides that retain bioactivity. The new peptides correspond to the inventors' discovery and determination that amino-terminal and carboxy-terminal functional domains are present in PTH(1-34) peptide. This aspect of the invention enables the development of agonists of PTH receptor activity by selectively manipulating the biochemical properties of each functional domain, separately or in combination. The general formula of the peptide compounds of this aspect of the invention is S-(L)ₙ-B, wherein S is a amino-terminal signaling functional domain; L is a linker molecule present n times, where n is an integer from 5-9, B is a carboxy-terminal functional domain of the peptide hormone, and wherein L is glycine.

As protein products, compounds and derivatives of the present invention are amenable to production by the technique of solution- or solid-phase peptide synthesis. The solid phase peptide synthesis technique, in particular, has been successfully applied in the production of human PTH and can be used for the production of S-(L)ₙ-B compounds of the invention or derivatives (for guidance, *see* Kimura *et al*., *supra*, and see Fairwell et al., Biochem. 22:2691 (1983)). Success with producing human PTH on a relatively large scale has been reported by Goud et al., in J. Bone Min. Res. 6(8):781 (1991). The synthetic peptide synthesis approach generally entails the use of automated synthesizers and appropriate resin as solid phase, to which is attached the C-terminal amino acid of the desired compounds or derivatives of the invention. Extension of the peptide in the N-terminal direction is then achieved by successively coupling a suitably protected form of the next desired amino acid, using either FMOC- or BOC-based chemical protocols typically, until synthesis is complete. Protecting groups are then cleaved from the peptide, usually simultaneously with cleavage of peptide from the resin, and the peptide is then isolated and purified using conventional techniques, such as by reversed phase HPLC using acetonitrile as solvent and tri-fluoroacetic acid as ion-pairing agent. Such procedures are generally described in numerous publications and reference may be made, for example, to Stewart and Young, "Solid Phase Peptide Synthesis," 2nd Edition, Pierce Chemical Company, Rockford, IL (1984). It will be appreciated that the peptide synthesis approach is required for production of S-(L)ₙ-B compounds of the invention and derivatives and variants thereof which incorporate amino acids that are not genetically encoded.

In one specific embodiment, this invention provides a biologically active peptide useful for the design of agonists for the PTH-1 or PTH-2 receptor comprising the compound S-(L)ₙ-B, wherein S is the signaling peptide PTH(I-9)(Ala Val Ser Glu Ile GIn Leu Met His (SEQ ID NO:1)); L is the linker molecule (Gly)₅; and B is a binding peptide PTH(15-31)(Leu Asn Ser Met Glu Arg Val Glu Trp Leu Arg Lys Lys Leu GIn Asp Val (SEQ ID NO:2)). The entire sequence being PG5: Ala Val Ser Glu Ile Gln Leu Met His Gly Gly Gly Gly Gly Leu Asn Ser Met Glu Arg Val Glu Trp Leu Arg Lys Lys Leu GIn Asp Val (SEQ ID NO:3)

The S-(L)ₙ-B compound represented by PTH(1-9)-(Gly)₅-PTH(15-31) may be altered by amino acid substitution in the S and B peptides. Substitutions may be with either L or D sterioisomers of any selected amino acid, natural or synthetic.

Suitable sites and substitutions in the S peptide (PTH(1-9)) include the following:
1) Position 3 (serine) to alanine: shown in the alanine scan of PTH(1-4) to enhance activity.
2) Position 3 (serine) to other small amino acids (e.g. Gly, Thr, Asn, Cys), (note, bulky amino acids at 3 impair activity, as shown in Cohen, F.E., et al., J. Biol. Chem. 266:1997-2004 (1991).
3) Position 1 (alanine) to other small or charged amino acids (e.g. Gly, Pro-Val Thr, Asp or Lys), include L and D sterioisomers, (note, residue 1 of PTH shown to be important for activation (Tregear, G.W., et al., Endocrinology 93:1349-1353 (1973)).
4) Position 2 (valine) to other hydrophobic or small amino acids (e.g. Ile, Gly, Thr,) (note, residue 2 ofPTH was shown earlier to be important for activation (Gardella, T.J., et al., J. Biol. Chem. 266:13141-13146 (1991)).
5) Position 4 (glutamate) to other charged or hydrophobic amino acids (e.g. Arg, Lys, Asp, Ile, Trp) (note, residue 4 of PTH was shown earlier to be important for activation (Gardella, T.J., et al., J. Biol. Chem. 266:13141-13146 (1991)).
6) Position 5 (isoleucine) to other charged or hydrophobic amino acids (e.g. Val. Met, Trp, His, Arg, Lys, Asp,) include L and D sterioisomers, (note, residue 5 of PTH was shown earlier to be important for activation and binding (Gardella, T.J., et al., J. Biol. Chem. 270:6584-6588 (1995); Gardella, T., et al., J. Biol. Chem. 271:19888-19893 (1996)).
7) Position 6 (glutamine) to other small, charged or hydrophobic amino acids (e.g. Ala, Leu, Arg, Lys, Asp,) (note, residue 6 data of PTH was shown earlier to be important for activation (Cohen, F.E., et al., J. Biol. Chem. 266:1997-2004 (1991)).

Suitable target sites and substitutions in the B peptide PTH(15-31)) include the following:
1) Position 19 (glutamate) to arginine: ER-19 substitution shown to improve binding of PTH(1-34) analogs (Gardella, T.J., et al., J. Biol. Chem. 270:6584-6588 (1995), Takasu et al., Biochemistry 38:13453-13460 (1999)).
2) Position 22 (glutamate) to alanine: EA-22 substitution shown to improve binding of PTH(17-31) (unpublished alanine scan data).

The S-(L)ₙ-B compound represented by PTH(1-9)-(Gly)₅-PTH(15-31) may be altered by the substitution of any L or D amino acid stereoisomer, natural or synthetic. Such substitutions may be made in either the S or B peptide wherein no more than one, two, three, four or five amino acids are changed or substituted. The design and synthesis of such compounds are within the skill of those in the art.

As those skilled in the art would know, other subsititions may be made in PTH(1-9)-(Gly)₅-PTH(15-31) such that all nine residues of the S peptide and all seventeen residues of the B peptide are substituted in pursuit of agonists of PTH receptor activity.

The PTH-1 receptor is a member of the family B subgroup of G protein-coupled receptors, which also includes the receptors for calcitonin and secretin (Kolakowski, L. F., "GCRDb: A G-Protein-Coupled Receptor Database," Receptors and Channels 2:1-7 (1994)). Mutagenesis and crosslinking studies have indicated that multiple domains of these receptors contribute to ligand interaction, including the large amino-terminal extracellular domain, the extracellular loops and the transmembrane helices (Jüppner, H., et al., Endocrinology 134:879-884 (1994); Lee, C., et al., Mol. Endo. 9:1269-1278 (1995); Turner, P., et al., J. Bone Min. Res. 12(1):Abstract 121 (1997); Dautzenberg, F., et al., Proc. Natl. Acad Sci. 95:4941-4946 (1998); Holtmann, M., et al., J. Biol. Chem. 270:14394-14398 (1995); DeAlmeida, V. and Mayo, K., Mol. Endo. 12:750-765 (1998); Stroop, S., et al., Biochem. 34:1050-1057 (1994); Zhou, A., et al., Proc. Natl. Acad. Sci. USA 94:3644-3649 (1997); Bisello, A., et al., J. Biol. Chem. 273:22498-22505 (1998)). Studies using PTH/calcitonin chimeric receptors and hybrid ligands have suggested a general topology of the interaction in which the amino-terminal extracellular domain of the receptor recognizes the carboxyl-terminal binding domain of the ligand, while the "core" region ofthe receptor containing the seven transmembrane helices and connecting loops recognizes the amino-terminal signaling portion ofthe ligand (Bergwitz, C., et al., J. Biol. Chem. 271:26469-26472 (1996)). Similar conclusions were derived from earlier receptor chimera studies (Jüppner, H., et al., Endocrinology 134:879-884 (1994); Stroop, S., et al., Biochem. 34:1050-1057 (1994); Gardella, T.J., et al., Endocrinology 135:1186-1194 (1994)) and from recent crosslinking studies with photoreactive PTH analogs (Bisello, A., et al., J. Biol. Chem. 273:22498-22505 (1998); Mannstadt, M., et al., J. Biol. Chem. 273:16890-16896 (1998)). Some recognition determinants for the family B receptors have been identified in the amino-terminal extracellular domain, the extracellular loops and the transmembrane helices (Turner, P., et al., Single Mutations Allow the PTH-2 Receptor to Respond to PTHrP J. Bone Min. Res. 12, Supplement 1, Abstract #121 (1997), Dautzenberg, F., et al., Proc. Natl. Acad. Sci. 95:4941-4946 (1998), Holtmann, M., et al., J. Biol. Chem. 270:14394-14398 (1995), Gardella, T.J., et al., Endrocrinology 135:1186-1194 (1994); Bergwitz, C., et al., J. Biol. Chem. 272:28861-28868; Turner, P.R., et al., J. Biol. Chem. 271(16):9205-9208 (1996)). Thus, G protein coupled receptor ligand systems, and the B family in particular, function through similar ligand/receptor interactions as the PTH/PTH receptor system.

In accordance with another aspect of the present invention, substituents may be attached to the free amine of the N-terminal amino acid of S-(L)ₙ-B compounds of the invention or derivatives thereof by standard methods known in the art in the making of a bioactive peptide. For example, alkyl groups, e.g., C₁₋₁₂ alkyl, may be attached using reductive alkylation. Hydroxyalkyl groups, e.g. C₁₋₁₂ hydroxyalkyl, may also be attached using reductive alkylation wherein the free hydroxy group is protected with a t-butyl ester. Acyl groups, e.g., COE₁, may be attached by coupling the free acid, e.g., E₁COOH, to the free amino of the *N*-terminal amino acid. S-(L)ₙ-B compounds of the invention or derivatives thereof are described herein that alter secondary or tertiary structure, or stability of S-(L)ₙ-B compounds of the invention or derivatives thereof which still retain biological activity. Such derivatives might be achieved through lactam cyclization, disulfide bonds, or other means known to a person of ordinary skill in the art.

In the above general structure, S-(L)ₙ-B, L is an amino acid residue (L)ₙ, wherein n is an integer from 5 to 9 and L is glycine.

Other linker molecules will be known to those skilled in the art and may be utilized for (L)ₙ in the general format S-(L)ₙ-B for the making of bioactive peptides.

### 3. Screen for S-(L)ₙ-B Activity on PTH Receptor

Functional characterization of the biological properties of the S-(L)ₙ-B compounds of the invention and derivatives thereof may be performed by bioassays that measure ligand-stimulated cAMP accumulation.

### A. Stimulation of Cyclic AMP Accumulation by S-(L)ₙ-B Compounds of the Invention

Intracellular cAMP accumulation is measured as described previously (Abou-Samra et al., J. Biol. Chem. 262:1129, 1986). Cells grown in 24-well plates are rinsed with culture medium containing 0.1% BSA and 2 mM IBMX. The cells are then incubated with an S-(L)ₙ-B compound or derivatives thereof for 60 min. at 21 ° C. The supernatant is removed and the cells immediately frozen by placing the whole plate in dry ice powder. Intracellular cAMP is extracted by thawing the cells in 1 ml of 50 mM HCI and analyzed by a specific radioimmunoassay using an anti-cAMP antibody (e.g., Sigma, St. Louis, Mo). A cAMP analog (2'-O-monosuccinyl-adenosine 3':5'-cyclic monophosphate tyrosyl methyl ester, obtained from Sigma) which is used a tracer for cAMP is iodinated by the chloramine T method. Free iodine is removed by adsorbing the iodinated cAMP analog onto a C18 Sep-pak cartridge (Waters, Milford, Mass.). After washing with dH₂O, the iodinated cAMP analog is eluted from the Sep-pak Cartridge with 40% acetonitrille (ACN) and 0.1% trifluoroacetic acid (TFA). The iodinated cAMP analog is lyophilized, reconstituted in 1 ml 0.1% TFA, and injected into a C18 reverse phase HPLC column (Waters). The column is equilibrated with 10% ACN in 0.1% TFA, and eluted with gradient of 10-30% ACN in 0.1 % TFA. This allows separation of the mono-iodinated cAMP analog from the non-iodinated cAMP analog. The tracer is stable for up to 4 months when stored at - 20°C. The standard used for the assay, adenosine 3':5'-cyclic monophosphate, may be purchased from Sigma. Samples (1-10 82 1 of HCl extracts) or standards (0.04-100 fmol/tube) are diluted in 50 mM Na-acetate (pH 5.5), and acetylated with 10 µl of mixture of triethylamine and acetic anhydride (2:1 vol:vol). After acetylation, cAMP antiserum (100 µl) is added from a stock solution (1:4000) made in PBS (pH 7.4), 5 mM EDTA and 1% normal rabbit serum. The tracer is diluted in PBS (pH 7.4) with 0.1% BSA, and added (20,000 cpm/tube). The assay is incubated at 4°C. overnight. The bound tracer is precipitated by adding 100 µl of goat anti-rabbit antiserum (1:20 in PBS) and 1 ml of 7% polyethyleneglycol (MW 5000-6000), centrifuging at 2000 rpm for 30 min. at 4°C. The supernatant is removed and the bound radioactivity is counted in a gamma-counter (Micromedic). To compute the cAMP data, logit calculations are performed in Excel spreadsheets. Typically, the assay sensitivity is 0.1 fmol/tube, and the standard concentration that displaces 50% of tracer is 5 fmol/tube.

### B. Binding of S-(L)ₙ-B Compounds of the Invention or Derivatives Thereof to Cloned Receptors Expressed on COS Cells

In addition to the cAMP accumulation assay described below, it is possible that S-(L)ₙ-B compounds of the invention or derivatives thereof may also be iodinated and used in a radioreceptor-based assay in transiently transfected COS cells. COS-7 cells are grown in 15 cm plates in DMEM, 10% heat-inactivated FBS, 10 mg/L gentamycin until 80-90% confluent. Twenty-four hours after transfection by the DEAE/Dextran method (Sambrook *et al., supra*), with 1-2µg of plasmid DNA, the cells are trypsinized and replated in multiwell plastic dishes (16 or 35 mm diameter, Costar, Cambridge, Mass.) at a cell concentration of 5 x 10⁴ cells/cm². Cell number increased only slightly after transfection. After continuing culture for another 48 h, radioreceptor assays are performed. The culture medium is replaced with buffer containing 50 mM Tris-HCL (pH 7.7), 100 mM NaCl, 2 mM CaCl₂, 5 mM KCL, 0.5% heat-inactivated fetal bovine serum (GIBCO), and 5% heat-inactivated horse serum (KC Biological Inc., Lenexa, Kans.) immediately before studies are initiated. Unless otherwise indicated, studies are conducted with cells incubated in this buffer at 15°C. for 4 h with 4 x 10⁵ cpm/ml (9.6 x 10⁻¹¹ M) of ¹²⁵I-labeled [Ala¹]PTH(1-14) amide or ¹²⁵I-labeled [Nle⁸PTH(1-14). Alternatively, more convention radioligands such as [nel8,21,Tyr34]-rPTH(1-34)NH₂ may also be used.

### 4. Vectors, Host Cells, and Recombinant Expression.

Vectors are described herein that comprise a S-(L)ₙ-B polynucleotide of the present invention, i.e. polynucleotides that encode the polypeptides of the invention. Such polynucleotide sequences are easily designed by those skilled in the art using the S-(L)ₙ-B peptide sequences provided herein. Host cells which are genetically engineered with vectors described herein may be used in the production of S-(L)ₙ-B polypeptides of the invention by recombinant techniques. Cell-free translation systems can also be employed to produce such proteins using RNAs derived from the DNA constructs of present invention.

For recombinant production, host cells can be genetically engineered to incorporate expression systems or portions thereof for S-(L)ₙ-B polynucleotides of the present invention. Introduction of polynucleotides into host cells can be effected by methods described in many standard laboratory manuals, such as Davis et al., Basic Methods in Molecular Biology (1986) and Sambrook et al., Molecular Cloning: A Laboratory Manual, 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as *streptococci, staphylococci, E. coli, Streptomyces* and *Bacillus subtilis* cells; fungal cells, such as yeast cells and Aspergillus cells; insect cells such as Drosophila S2 and Spodoptera Sf9 cells; animal cells such as CHO, COS, HeLa, C127, 3T3, BHK, 293 and Bowes melanoma cells; and plant cells.

A great variety of expression systems can be used. Such systems include, among others, chromosomal, episomal and virus-derived systems, *e.g.*, vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses, and retroviruses, and vectors derived from combinations thereof, such as those derived from plasmid and bacteriophage genetic elements, such as cosmids and phagemids. The expression systems may contain control regions that regulate as well as engender expression. Generally, any system or vector suitable to maintain, propagate or express polynucleotides to produce a polypeptide in a host may be used. The appropriate nucleotide sequence may be inserted into an expression system by any of a variety of well-known and routine techniques, such as, for example, those set forth in Sambrook *et al.,* Molecular Cloning: A Laboratory Manual (*supra*).

RNA vectors may also be utilized for the expression of the nucleic acids encoding S-(L)ₙ-B compounds or derivatives thereof disclosed in this invention. These vectors are based on positive or negative strand RNA viruses that naturally replicate in a wide variety of eukaryotic cells (Bredenbeek, P.J. & Rice, C.M., Virology 3: 297-310, 1992). Unlike retroviruses, these viruses lack an intermediate DNA life-cycle phase, existing entirely in RNA form. For example, alpha viruses are used as expression vectors for foreign proteins because they can be utilized in a broad range of host cells and provide a high level of expression; examples of viruses of this type include the Sindbis virus and Semliki Forest virus (Schlesinger, S., TIBTECH 11:18-22, 1993; Frolov, I., et al., Proc. Natl. Acad. Sci. (USA) 93: 11371-11377, 1996). As exemplified by Invitrogen's Sinbis expression system, the investigator may conveniently maintain the recombinant molecule in DNA form (pSinrep5 plasmid) in the laboratory, but propagation in RNA form is feasible as well. In the host cell used for expression, the vector containing the gene of interest exists completely in RNA form and may be continuously propagated in that state if desired.

For secretion of the translated protein into the lumen of the endoplasmic reticulum, into the periplasmic space or into the extracellular environment appropriate secretion signals may be incorporated into the desired S-(L)ₙ-B polypeptide. These signals may be endogenous to the polypeptide or they may be heterologous signals.

The expression of a DNA sequence requires that the DNA sequence be "operably linked" to DNA sequences which contain transcriptional and translational regulatory information. An operable linkage is a linkage in which the control or regulatory DNA sequences and the DNA sequence sought to be expressed are connected in such a way as to permit gene expression. The precise nature of the "control regions" needed for gene expression may vary from organism to organism, but shall in general include a promoter region which, in prokaryotic cells, contains both the promoter (which directs the initiation ofRNA transcription) as well as DNA sequences which, when transcribed into RNA, will signal the initiation of protein synthesis. Regulatory regions in eukaryotic cells will in general include a promoter region sufficient to direct the initiation of RNA synthesis.

Two DNA sequences are said to be operably linked if the nature of the linkage between the two DNA sequences does not (1) result in the introduction of a frameshift mutation, (2) interfere with the ability of the promoter region sequence to direct the transcription ofthe fusion protein-encoding sequence or (3) interfere with the ability of the fusion protein-encoding sequence to be transcribed by the promoter region sequence. Thus, a promoter region would be operably linked to a DNA sequence if the promoter were capable of transcribing that DNA sequence.

The joining of various DNA fragments, to produce the expression vectors described herein is performed in accordance with conventional techniques, employing blunt-ended or staggered-ended termini for ligation, restriction enzyme digestion to provide appropriate termini, filling in of cohesive ends as appropriate, alkali and phosphatase treatment to avoid undesirable joining, and ligation with appropriate ligates. In the case of a fusion protein, the genetic construct encodes an inducible promoter which is operably linked to the 5' gene sequence of the fusion protein to allow efficient expression of the fusion protein.

To express S-(L)ₙ-B compounds of the invention or a derivative thereof in a prokaryotic cell (such as, for example, *E*. *coli, B. subtilis, Pseudomonas, Streptomyces,* etc.), it is necessary to operably link the SEQ ID NO: 1-encoding DNA sequence to a functional prokaryotic promoter. Such promoters may be either constitutive or, more preferably, regulatable (*i.e*., inducible or derepressible). Examples of constitutive promoters include the int promoter of bacteriophage λ, the bla promoter of the β-lactamase gene of pBR322, and the CAT promoter of the chloramphenicol acetyl transferase gene of pBR325, etc. Examples of inducible prokaryotic promoters include the major right and left promoters of bacteriophage λ, (PL and PR), the trp, recA. lacZ. lacI. and gal promoters of *E*. *coli,* the α-amylase (Ulmanen, I. et al., J. Bacteriol. 162:176-182 (1985)), and the σ-28-specific promoters of *B*. *subtilis* (Gilman, M. Z. et al., Gene 32:11-20 (1984)), the promoters of the bacteriophages of *Bacillius* (Gryczan, T. J., In: The Molecular Biology of the Bacilli, Academic Press, Inc., NY (1982)), and *Streptomyces* promoters (Ward, J. M. et al., Mol. Gen. Genet. 203:468-478 (1986)). Prokaryotic promoters are reviewed by Glick, B. R., J. Ind. Microbiol. 1:277-282 (1987); Cenatiempo, Y., Biochimie 68:505-516 (1986)); and Gottesman, S., Ann. Rev. Genet. 18:415-442 (1984)).

The preferred prokaryotic promoter is the *E. coli* trp promoter, which is inducible with indole acrylic acid.

If expression is desired in a eukaryotic cell, such as yeast, fungi, mammalian cells, or plant cells, then it is necessary to employ a promoter capable of directing transcription in such a eukaryotic host. Preferred eukaryotic promoters include the promoter of the mouse metallothionein I gene (Hamer, D. et al., J. Mol. Appl. Gen. 1:273-288 (1982)); the TK promoter of Herpes virus (McKnight, S., Cell 31:355-365 (1982)); the SV40 early promoter (Benoist, C., et al., Nature (London) 290:3 04-310 (1981)); and the yeast gal4 gene promoter (Johnston, S. A., et al., Proc. Natl. Acad. Sci. (USA) 79:6971-6975 (1982); Silver, P. A., et al., Proc. Natl. Acad. Sci. (USA) 81:5951-5955 (1984)).

Preferably, the introduced gene sequence will be incorporated into a plasmid or viral vector capable of autonomous replication in the recipient host. Any of a wide variety of vectors may employed for this purpose. Factors of importance in selecting a particular plasmid or viral vector include: the ease with which recipient cells that contain the vector may be recognized and selected from those recipient cells which do not contain the vector; the number of copies of the vector which are desired in a particular host; and whether it is desirable to be able to "shuttle" the vector between host cells of different species.

Preferred prokaryotic vectors include plasmids such as those capable of replication in E. coli (such as, for example, pBR322, ColEl, pSC101, pACYC 184, πVX. Such plasmids are, for example, disclosed by Maniatis, T., et al., In: Molecular Cloning, A Laboratory Manual, Cold Spring Harbor Press, Cold Spring Harbor, NY (1982)). Preferred plasmid expression vectors include the pGFP-1 plasmid described in Gardella et al., J. Biol. Chem. 265:15854-15859 (1989), or a modified plasmid based upon one of the pET vectors described by Studier and Dunn, Methods in Enzymology 185: 60-89 (1990). *Bacillus* plasmids include pC194, pC221, pT127, etc. Such plasmids are disclosed by Gryczan, T. In: The Molecular Biology of the Bacilli, Academic Press, NY pp. 307-329 (1982). Suitable *Streptomyces* plasmids include pIJIOI (Kendall, K. J. et al., J. Bacteriol. 169:4177-4183 (1987)), and streptomyces bacteriophages such as φC31 (Chater, K. F. et al., In: Sixth International Symposium on Actinomycetales Biology, Akademiai Kaido, Budapest, Hungary, pp. 45-54 (1986)). Pseudomonas plasmids are reviewed by John, J. F. et al., Rev. Infect. Dis. 8:693-704 (1986)), and Izaki, K., Jon. J. Bacteriol. 33:729-742 (1978)).

Preferred eukaryotic expression vectors include, without limitation, BPV, vaccinia, 2-micron circle etc. Such expression vectors are well known in the art (Botstein, D., et al., Miami Wntr. Symp. 19:265-274 (1982); Broach, J. R., In: The Molecular Biology of the Yeast Saccharomyces: Life Cycle and Inheritance, Cold Spring Harbor Laboratory, Cold Spring Harbor, NY pp. 445-470 (1981); Broach, J. R., Cell 28:203-204 (1982); Bollon, D. P., et al., J. Clin. Hematol. Oncol. 10:39-48 (1980); Maniatis, T., In: Cell Biology: A Comprehensive Treatise, Vol. 3, Gene Expression, Academic Press, NY, pp. 563-608 (1980)).

In addition to microorganisms, cultures of cells derived from multicellular organisms may also be used as hosts. In principle, any such cell culture is workable, whether from vertebrate or invertebrate cellular sources. Interest, however, has been greater with cells from vertebrate sources. Examples of useful vertebrate host cell lines are VERO and HeLa cells, Chinese hamster ovary (CHO) cell lines, WI38, BHK, COS-7, and MDCK cell lines. Expression vectors for such cells ordinarily include (if necessary) an origin of replication, a promoter located in front of or upstream to the gene to be expressed, along with any necessary ribosome binding sites, RNA splice sites, polyadenylation site, and transcriptional terminator sequences.

For use in mammalian cells, the control functions on the expression vectors are often provided by viral material. For example, commonly used promoters are derived from polyoma, Adenovirus 2, Simian Virus 40 (SV40) and cytomegalovirus. The early and late promoters of SV40 virus are particularly useful because both are obtained easily from the virus as a fragment which also contains the SV40 vial origin of replication (Fiers *et al., Nature 273*:113 (1978)).

An origin of replication may be provided either by construction of the vector to include an exogenous origin, such as may be derived from SV40 or other viral (*e.g*. Polyoma, Adeno, VSV, BPV) source or may be provided by the host cell chromosomal replication mechanism. If the vector is integrated into the host cell chromosome, the latter is often sufficient.

If cells without formidable cell membrane barriers are used as host cells, transfection is carried out by the calcium phosphate precipitation method as described by Graham and Van der Erb, Virology 52:546 (1978). However, other methods for introducing DNA into cells, such as by nuclear injection or by protoplast fusion may also be used. In the case of gene therapy, the direct naked plasmid or viral DNA injection method, with or without transfection-facilitating agents such as, without limitation, liposomes, provides an alternative approach to the current methods of *in vivo* or *in vitro* transfection of mammalian cells. If prokaryotic cells or cells which contain substantial cell wall constructions are used, the preferred method of transfection is calcium treatment, using calcium chloride as described in Cohen et al., Proc. Natl. Acad. Sci. USA 69:2110 (1972).

### 5. Utility and Administration of S-(L)ₙ-B Compounds of the Invention or Derivatives Thereof

S-(L)ₙ-B Compounds of the invention or derivatives thereof are useful for the prevention and treatment of a variety of mammalian conditions manifested by loss of bone mass. In particular, the compounds ofthis invention are indicated for the prophylaxis and therapeutic treatment of osteoporosis and osteopenia in humans. Furthermore, the compounds of this invention are indicated for the prophylaxis and therapeutic treatment of other bone diseases. The compounds of this invention are indicated for the prophylaxis and therapeutic treatment of hypoparathyroidism. Finally, the compounds of this invention are indicated for use as agonists for fracture repair and as antagonists for hypercalcemia.

In general, S-(L)ₙ-B compounds or derivatives thereof of this invention, or salts thereof, are administered in amounts between about 0.01 and 1 µg/kg body weight per day, preferably from about 0.07 to about 0.2 µg/kg body weight per day. For a 50 kg human female subject, the daily dose of biologically active compounds of SEQ ID NO: 1 or derivatives thereof is from about 0.5 to about 50 µgs, preferably from about 3.5 to about 10 µgs. In other mammals, such as horses, dogs, and cattle, higher doses may be required. This dosage may be delivered in a conventional pharmaceutical composition by a single administration, by multiple applications, or via controlled release, as needed to achieve the most effective results, preferably one or more times daily by injection. For example, this dosage may be delivered in a conventional pharmaceutical composition by nasal insufflation.

The selection of the exact dose and composition and the most appropriate delivery regimen will be influenced by, *inter alia,* the pharmacological properties of the selected S-(L)ₙ-B compounds of the invention or derivatives thereof, the nature and severity of the condition being treated, and the physical condition and mental acuity of the recipient.

Representative preferred delivery regimens include, without limitation, oral, parenteral (including subcutaneous, transcutaneous, intramuscular and intravenous), rectal, buccal (including sublingual), transdermal, and intranasal insufflation.

Pharmaceutically acceptable salts retain the desired biological activity of the S-(L)ₙ-B compounds of the invention or derivatives thereof without toxic side effects. Examples of such salts are (a) acid addition salts formed with inorganic acids, for example hydrochloric acid, hydrobromic acid, sulfuric acid, phosphoric acid, nitric acid and the like; and salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, pamoic acid, alginic acid, polyglutamic acid, naphthalenesulfonic acids, naphthalene disulfonic acids, polygalacturonic acid and the like; (b) base addition salts formed with polyvalent metal cations such as zinc, calcium, bismuth, barium, magnesium, aluminum, copper, cobalt, nickel, cadmium, and the like; or with an organic cation formed from N,N'-dibenzylethylenediamine or ethylenediamine; or (c) combinations of (a) and (b), e.g., a zinc tannate salt and the like.

Pharmaceutical compositions are described herein comprising as an active ingredient S-(L)ₙ-B compounds of or derivatives thereof of the present invention, or pharmaceutically acceptable salt thereof, in admixture with a pharmaceutically acceptable, non-toxic carrier. As mentioned above, such compositions may be prepared for parenteral (subcutaneous, transcutaneous, intramuscular or intravenous) administration, particularly in the form of liquid solutions or suspensions; for oral or buccal administration, particularly in the form of tablets or capsules; for rectal, transdermal administration; and for intranasal administration, particularly in the form of powders, nasal drops or aerosols.

The S-(L)ₙ-B compositions may conveniently be administered in unit dosage form and may be prepared by any of the methods well-known in the pharmaceutical art, for example as described in Remington's Pharmaceutical Sciences, 17th ed., Mack Publishing Company, Easton, Pa., (1985). Formulations for parenteral administration may contain as excipients sterile water or saline, alkylene glycols such as propylene glycol, polyalkylene glycols such as polyethylene glycol, oils of vegetable origin, hydrogenated naphthalenes and the like. For oral administration, the formulation can be enhanced by the addition of bile salts or acylcarnitines. Formulations for nasal administration may be solid and may contain excipients, for example, lactose or dextran, or may be aqueous or oily solutions for use in the form of nasal drops or metered spray. For buccal administration typical excipients include sugars, calcium stearate, magnesium stearate, pregelatinated starch, and the like.

When formulated for the most preferred route of administration, nasal administration, the absorption across the nasal mucous membrane may be enhanced by surfactant acids, such as for example, glycocholic acid, cholic acid, taurocholic acid, ethocholic acid, deoxycholic acid, chenodeoxycholic acid, dehydrocholic acid, glycodeoxycholic acid, cyclodextrins and the like in an amount in the range between about 0.2 and 15 weight percent, preferably between about 0.5 and 4 weight percent, most preferably about 2 weight percent.

Delivery ofthe S-(L)ₙ-B compounds ofthe present invention to the subject over prolonged periods of time, for example, for periods of one week to one year, may be accomplished by a single administration of a controlled release system containing sufficient active ingredient for the desired release period. Various controlled release systems, such as monolithic or reservoir-type microcapsules, depot implants, osmotic pumps, vesicles, micelles, liposomes, transdermal patches, iontophoretic devices and alternative injectable dosage forms may be utilized for this purpose. Localization at the site to which delivery of the active ingredient is desired is an additional feature of some controlled release devices, which may prove beneficial in the treatment of certain disorders.

One form of controlled release formulation contains the polypeptide or its salt dispersed or encapsulated in a slowly degrading, non-toxic, non-antigenic polymer such as copoly(lactic/glycolic) acid, as described in the pioneering work of Kent, Lewis, Sanders, and Tice, U.S. Pat. No. 4,675,189. The compounds or, preferably, their relatively insoluble salts, may also be formulated in cholesterol or other lipid matrix pellets, or silastomer matrix implants. Additional slow release, depot implant or injectable formulations will be apparent to the skilled artisan. See, for example, Sustained and Controlled Release Drug Delivery Systems, J. R. Robinson ed., Marcel Dekker, Inc., New York, 1978, and R. W. Baker, Controlled Release of Biologically Active Agents, John Wiley & Sons, New York, 1987.

Like PTH, the S-(L)ₙ-B variants may be administered in combination with other agents useful in treating a given clinical condition. When treating osteoporosis and other bone-related disorders for example, the S-(L)ₙ-B variants may be administered in conjunction with a dietary calcium supplement or with a vitamin D analog (*see* U.S. Pat. No. 4,698,328). Alternatively, the S-(L)ₙ-B variant may be administered, preferably using a cyclic therapeutic regimen, in combination with bisphosphonates, as described for example in U.S. Pat. No. 4,761,406, or in combination with one or more bone therapeutic agents such as, without limitation, calcitonin and estrogen.

### V. Receptor-Signaling Activities of S-(L)ₙ-B Compounds of the Invention or Derivatives Thereof

A crucial step in the expression of hormonal action is the interaction of hormones with receptors on the plasma membrane surface of target cells. The formation ofhormone-receptor complexes allows the transduction ofextracellular signals into the cell to elicit a variety of biological responses.

### A. Screening for PTH-1 Receptor Antagonists and Agonists

S-(L)ₙ-B polypeptides of the invention may be screened for their agonistic or antagonistic properties using the cAMP accumulation assay. Cells expressing PTH-1 receptor on the cell surface are incubated with native PTH(1-84) for 5-60 minutes at 37°C., in the presence of 2 mM IBMX (3-isobutyl-1-methyl-xanthine, Sigma, St. Louis, MO). Cyclic AMP accumulation is measured by specific radio-immunoassay, as described above. An S-(L)ₙ-B compound of the invention or a derivative thereof that competes with native PTH(1-84) for binding to the PTH-1 receptor, and that inhibits the effect of native PTH(1-84) on cAMP accumulation, is considered a competitive antagonist. Such a compound would be useful for treating hypercalcemia.

Conversely, an S-(L)ₙ-B compound of the invention or a derivative thereof that does not compete with native PTH(1-84) for binding to the PTH-1 receptor, but which still prevents native PTH(1-84) activation of cAMP accumulation (presumably by blocking the receptor activation site) is considered a non-competitive antagonist. Such a compound would be useful for treating hypercalcemia.

An S-(L)ₙ-B compound of the invention or a derivative thereof that competes with native PTH(1-84) for binding to the PTH-1 receptor, and which stimulates cAMP accumulation in the presence or absence of native PTH(1-84) is a competitive agonist. An S-(L)ₙ-B compound of the invention or a derivative thereof that does not compete with native PTH(1-84) for binding to the PTH-1 receptor but which is still capable of stimulating cAMP accumulation in the presence or absence of native PTH(1-84), or which stimulates a higher cAMP accumulation than that observed by a compound of SEQ ID NO: 1 or a derivative thereof alone, would be considered a non-competitive agonist.

### 6. Therapeutic Uses of S-(L)ₙ-B Compounds of the Invention or Derivatives Thereof

Some forms of hypercalcemia and hypocalcemia are related to the interaction between PTH and PTHrP and the PTH-1 and PTH-2 receptors. Hypercalcemia is a condition in which there is an abnormal elevation in serum calcium level; it is often associated with other diseases, including hyperparathyroidism, osteoporosis, carcinomas of the breast, lung and prostate, epidermoid cancers of the head and neck and of the esophagus, multiple myeloma, and hypernephroma. Hypocalcemia, a condition in which the serum calcium level is abnormally low, may result from a deficiency of effective PTH, *e.g.*, following thyroid surgery.

Nucleic acids of the invention which encode an S-(L)ₙ-B compound of the invention or derivatives thereof may also be linked to a selected tissue-specific promoter and/or enhancer and the resultant hybrid gene introduced, by standard methods (*e.g*., as described by Leder et al., U.S. Pat. No. 4,736,866, into a non-human animal embryo at an early developmental stage (*e.g*., the fertilized oocyte stage), to produce a transgenic animal which expresses elevated levels of an S-(L)ₙ-B compound of the invention or derivatives thereof in selected tissues (*e.g.*, the osteocalcin promoter for bone). Such promoters are used to direct tissue-specific expression of compounds of SEQ ID NO: 1 or derivatives thereof in the transgenic animal.

In addition, any other amino-acid substitutions of a nature, which do not destroy the ability of the an S-(L)ₙ-B compound of the invention to antagonize or agonize the PTH-1/PTH-2 receptor (as determined by assays known to the skilled artisan and discussed below), are described herein.

By "agonist" is intended a ligand capable of enhancing or potentiating a cellular response mediated by the PTH-1 receptor. By "antagonist" is intended a ligand capable of inhibiting a cellular response mediated by the PTH-1 receptor. Whether any candidate "agonist" or "antagonist" can enhance or inhibit such a cellular response can be determined using art-known protein ligand/receptor cellular response or binding assays, including those described elsewhere in this application.

Further described herein is the use of an therapeutically effective amount of an S-(L)ₙ-B compound of the invention or a derivative thereof for treating a medical disorder that results from altered or excessive action of the PTH-1 receptor.

A patient who is suspected of having a disorder resulting from altered action of the PTH-1 receptor may be treated with a medicament comprising an S-(L)ₙ-B compound of the invention or derivatives thereof which are selective antagonists of the PTH-1 receptor. Such antagonists include an S-(L)ₙ-B compound of the invention or derivatives thereof which have been determined (by the assays described herein) to interfere with PTH-1 receptor-mediated cell activation or other derivatives having similar properties.

To administer the antagonist, the appropriate an S-(L)ₙ-B compound of the invention or a derivative thereof is used in the manufacture of a medicament, generally by being formulated in an appropriate carrier or excipient such as, *e.g.*, physiological saline, and preferably administered intravenously, intramuscularly, subcutaneously, orally, or intranasally, at a dosage that provides adequate inhibition of an S-(L)ₙ-B compound of the invention or a derivative thereof binding to the PTH-1 receptor. Typical dosage would be 1 ng to 10 mg of the peptide per kg body weight per day.

In accordance with yet a further aspect of the invention, there is provided the use of an S-(L)ₙ-B compound of the invention or a derivative thereof, in the manufacture of a medicament for treating osteoporosis.

Similar dosages and administration as described above for the PTH/PTHrP antagonist, may be used for administration of a PTH/PTHrP agonist, *e.g.*, for treatment of conditions such as osteoporosis, other metabolic bone disorders, and hypoparathyroidism and related disorders.

### Example 1

### Construction of PTH Functional Domain Conjugate Peptides

PTH functional conjugate peptides may be constructed using well known methods in the art of molecular biology. The inventors constructed the ligand amino-terminal fragment used herein, PTH(1-9), based upon other studies related to the first 14 amino acids of native human PTH. To optimize activity of this fragment, the inventors replaced the serine at position one by alanine; a substitution which corresponds to the amino acid found at position 1 in rat and bovine PTH, as well as in all PTHrP molecules reported so far (human, bovine, dog, rat, mouse, chicken). This change results in a measurable increase in bioactivity over the background level of bioactivity of the native PTH(1-14) peptide. The C-terminal residue of this new peptide, herein called [Ala¹] PTH(1-14), is amidated. The amino-terminal functional domain of PTH(1-9) is based on this sequence. The carboxy-terminal functional domain of PTH(15-31) (Leu Asn Ser Met Glu Arg Val Glu Trp Leu Arg Lys Lys Leu Gln Asp Val) (SEQ ID NO:2) is detailed herein, as well as other variations used in the construction of the peptides. Peptides were created synthetically following procedures well known in the art. Alternatively, the peptides may be created via recombinant DNA techniques by reverse translation. Depending on the organism being used, codon usage charts are helpful in determining the appropriate code. For an example see Figure 8, wherein the DNA and protein sequences of PG5, PG7 and PG9 are detailed.

### Example 2

### Accumulation of cAMP in Cells Exposed to PTH Functional Domain Conjugate Peptides

In order to screen for agonist activity, functional domain conjugate peptides of the invention were utilized in *in vitro* assays designed to measure cellular response via cAMP accumulation. Intracellular cAMP accumulation is measured as described previously (Abou-Samra et al., J. Biol. Chem. 262:1129, 1986).

Cells grown in 24-well plates are rinsed with culture medium containing 0.1% BSA and 2 mM IBMX. The cells are then incubated with an S-(L)ₙ-B compound or derivatives thereof for 60 min. at 21 °C. The supernatant is removed and the cells immediately frozen by placing the whole plate in dry ice powder. Intracellular cAMP is extracted by thawing the cells in 1 ml of 50 mM HCl and analyzed by a specific radioimmunoassay using an anti-cAMP antibody (e.g., Sigma, St. Louis, Mo). A cAMP analog (2'-O-monosuccinyl-adenosine 3':5'-cyclic monophosphate tyrosyl methyl ester, obtained from Sigma) which is used a tracer for cAMP is iodinated by the chloramine T method. Free iodine is removed by adsorbing the iodinated cAMP analog onto a C 18 Sep-pak cartridge (Waters, Milford, Mass.). After washing with dH₂O, the iodinated cAMP analog is eluted from the Sep-pak Cartridge with 40% acetonitrille (ACN) and 0.1% trifluoroacetic acid (TFA). The iodinated cAMP analog is lyophilized, reconstituted in 1 ml 0.1 % TFA, and injected into a C18 reverse phase HPLC column (Waters). The column is equilibrated with 10% ACN in 0.1% TFA, and eluted with gradient of 10-30% ACN in 0.1% TFA. This allows separation of the mono-iodinated cAMP analog from the non-iodinated cAMP analog. The tracer is stable for up to 4 months when stored at - 20°C. The standard used for the assay, adenosine 3':5'-cyclic monophosphate, may be purchased from Sigma. Samples (1-10 821 of HCl extracts) or standards (0.04-100 fmol/tube) are diluted in 50 mM Na-acetate (pH 5.5), and acetylated with 10 µl of mixture of triethylamine and acetic anhydride (2:1 vol:vol). After acetylation, cAMP antiserum (100 µl) is added from a stock solution (1:4000) made in PBS (pH 7.4), 5 mM EDTA and 1% normal rabbit serum. The tracer is diluted in PBS (pH 7.4) with 0.1% BSA, and added (20,000 cpm/tube). The assay is incubated at 4°C. overnight. The bound tracer is precipitated by adding 100 µl of goat anti-rabbit antiserum (1:20 in PBS) and 1 ml of 7% polyethyleneglycol (MW 5000-6000), centrifuging at 2000 rpm for 30 min. at 4°C. The supernatant is removed and the bound radioactivity is counted in a gamma-counter (Micromedic). To compute the cAMP data, logit calculations are performed in Excel spreadsheets. Typically, the assay sensitivity is 0.1 fmol/tube, and the standard concentration that displaces 50% of tracer is 5 fmol/tube.

Results of cAMP accumulation experiments are presented in Figures 3 and 4. Figure 3 presents data for the total accumulation of cAMP, while Figure 4 presents a PTH peptide dose response curve for cAMP accumulation. Importantly, the PTH functional domain peptides PG5, PG7 and PG9 all demonstrate increased levels of cAMP accumulation as compared to basal levels in un-stimulated cells.

### Example 3

### Alanine Scans of PTH (1-14) and PTH (17-31)

In order to determine the bioactivity of each amino acid residue in amino-terminal PTH (1-9) signaling peptide and carboxy-terminal PTH(17-31) binding peptide, alanine was substituted for each individual amino acid residue of the two functional domain peptides. The synthesized peptides were used to determine bioactivity of the native amino acid residue by either by cAMP accumulation or competitive binding as described herein. Results for PTH (1-9) are presented in Figure 5, the alanine scan of PTH (1-14). Results for PTH(17-31) are presented in Figure 6. The results are useful for the design of agonists and antagonists of PTH receptor function and may be used in the construction of S-(L)ₙ-B peptides of the invention.

The foregoing specification, including the specific embodiments and examples, is intended to be illustrative of the present invention and is not to be taken as limiting.

## Claims

1. A compound of the structure or formula S-(L)ₙ-B wherein:
a) S is an amino terminal signaling functional domain of PTH consisting of PTH(1-9):Ala Val Ser Glu Ile GIn Leu Met His (SEQ ID NO: 1);
b) L is a linker molecule present n times, wherein n is 5-9;
c) B is a C-terminal binding portion of PTH (1-34) consisting of a PTH (15-31):(Leu Asn Ser Met Glu Arg Val Glu Trp Leu Arg Lys Lys Leu Gln Asp VaI (SEQ ID NO:2); and wherein L is glycine.

2. The compound of claim 1, wherein said compound is an isolated polypeptide.

3. The isolated polypeptide of claim 2, wherein (L)ₙ is selected from the group consisting of Gly₅, Gly₇ and Gly₉.

4. The isolated polypeptide of claim 2 consisting of PG5: Ala Val Ser Glu Ile GIn Leu Met His Gly Gly Gly Gly Gly Leu Asn Ser Met Glu Arg Val Glu Trp Leu Arg Lys Lys Leu Gln Asp Val (SEQ ID NO:3).

5. The isolated polypeptide of claim 3 or claim 4 wherein said polypeptide is modified by addition of C₁₋₁₂ alkyl groups, the addition of C₁₋₁₂ hydroxyalkyl groups, the addition of acyl groups or lactam cyclization.

6. The isolated polypeptide as claimed in any one of claims 2-5, wherein said polypeptide induces intracellular CLAMP accumulation.

7. The isolated polypeptide of claim 2, encoded by the nucleic acid sequence of: SEQ ID NO:14.

8. An isolated nucleic acid sequence encoding the polypeptide of any one of claims 2-5.

9. The use of a polypeptide of any one of claims 2-5 in the manufacture of a medicament for treatment of mammalian hypercalcaemia or mammalian hypocalcaemia.

10. The use of a polypeptide of any one of claims 2-5 in the manufacture of a reagent for determining rates of bone reformation, bone resorption and/or bone remodeling.

11. The use of DNA encoding a polypeptide as claimed in any one of claims 2-5, in the manufacture of a medicament for use in the treatment of mammalian hypercalcaemia or mammalian hypocalcaemia, wherein said polypeptide is expressed *in vitro.*

12. The use of claim 9 or claim 11, wherein the condition to be treated is osteoporosis.

13. The use of claim 9, wherein the dose of said polypeptide is from about 0.01 µg/kg/day to about 1.0 µg/kg/day.

14. An in vitro method of increasing cAMP in a mammalian cell having PTH-1 receptors comprising contacting a cell with a sufficient amount of a polypeptide of any one of claims 2-4.

## Patentansprüche

1. Verbindung der Struktur oder Formel S- (L)ₙ-B, wobei:
a) S eine Amino-terminale funktionelle Signaldomäne von PTH, bestehend aus PTH(1-9): Ala Val Ser Glu Ile Gln Leu Met His (SEQ ID Nr. 1), ist;
b) L ein Linkermolekül ist, das n-mal vorhanden ist, wobei n 5-9 ist;
c) B ein C-terminaler Bindungsteil von PTH(1-34), bestehend aus einem PTH(15-31) : Leu Asn Ser Met Glu Arg Val Glu Trp Leu Arg Lys Lys Leu Gln Asp Val (SEQ ID Nr. 2), ist; und wobei L Glycin ist.

2. Verbindung nach Anspruch 1, wobei die Verbindung ein isoliertes Polypeptid ist.

3. Isoliertes Polypeptid nach Anspruch 2, wobei (L)ₙ ausgewählt ist aus der Gruppe bestehend aus Gly₅, Gly₇ und Gly₉.

4. Isoliertes Polypeptid nach Anspruch 2, bestehend aus PG5: Ala Val Ser Glu Ile Gln Leu Met His Gly Gly Gly Gly Gly Leu Asn Ser Met Glu Arg Val Glu Trp Leu Arg Lys Lys Leu Gln Asp Val (SEQ ID Nr. 3).

5. Isoliertes Polypeptid nach Anspruch 3 oder 4, wobei das Polypeptid durch Addition von C₁₋₁₂-Alkylgruppen, Addition von C₁₋₁₂-Hydroxyalkylgruppen, Addition von Acylgruppen oder durch Lactam-Cyclisierung modifiziert ist.

6. Isoliertes Polypeptid nach einem der Ansprüche 2 bis 5, wobei das Polypeptid eine intrazelluläre cAMP-Akkumulation induziert.

7. Isoliertes Polypeptid nach Anspruch 2, kodiert durch die Nukleinsäuresequenz von SEQ ID Nr. 14.

8. Isolierte Nukleinsäuresequenz, die das Polypeptid nach einem der Ansprüche 2 bis 5 kodiert.

9. Verwendung eines Polypeptids nach einem der Ansprüche 2 bis 5 bei der Herstellung eines Medikaments zur Behandlung von Hyperkalzämie bei Säugetieren oder Hypokalzämie bei Säugetieren.

10. Verwendung eines Polypeptids nach einem der Ansprüche 2 bis 5 bei der Herstellung eines Reagens zur Bestimmung der Grade an Knochenwiederaufbau, Knochenresorption und/oder Knochenremodellierung.

11. Verwendung einer DNA, die ein Polypeptid nach einem der Ansprüche 2 bis 5 kodiert, bei der Herstellung eines Medikaments zur Verwendung bei der Behandlung von Hyperkalzämie bei Säugetieren oder Hypokalzämie bei Säugetieren, wobei das Polypeptid *in vitro* exprimiert ist.

12. Verwendung nach Anspruch 9 oder 11, wobei der zu behandelnde Zustand Osteoporose ist.

13. Verwendung nach Anspruch 9, wobei die Dosierung des Polypeptids ungefähr 0,01 µg/kg/Tag bis ungefähr 1,0 µg/kg/Tag ist.

14. In-vitro-Verfahren zum Erhöhen des cAMP in einer Säugetier-Zelle mit PTH-1-Rezeptoren, umfassend das Kontaktieren einer Zelle mit einer ausreichenden Menge an einem Polypeptid nach einem der Ansprüche 2 bis 4.

## Revendications

1. Composé de structure ou de formule S-(L)ₙ-B, dans laquelle :
a) S représente un domaine fonctionnel amino-terminal de signalisation de la PTH constitué d'une PTH(1-9) : Ala Val Ser Glu Ile Gln Leu Met His (SEQ ID n° 1) ;
b) L représente une molécule de liaison présente n fois, dans laquelle n vaut 5 à 9 ;
c) B représente une partie de liaison C-terminale de la PTH(1-34) constituée d'une PTH (15-31) : Leu Asn Ser Met Glu Arg Val Glu Trp Leu Arg Lys Lys Leu Gln Asp Val (SEQ ID n° 2) ; et dans laquelle L représente la glycine.

2. Composé selon la revendication 1, dans laquelle ledit composé est un polypeptide isolé.

3. Polypeptide isolé selon la revendication 2, dans laquelle (L)ₙ est choisi dans le groupe constitué de Gly₅, Gly₇ et GIy₉.

4. Polypeptide isolé selon la revendication 2, constitué d'une PG5 : Ala Val Ser Glu Ile Gln Leu Met His Gly Gly Gly Gly Gly Leu Asn Ser Met Glu Arg Val Glu Trp Leu Arg Lys Lys Leu Gln Asp Val (SEQ ID n° 3).

5. Polypeptide isolé selon la revendication 3 ou la revendication 4, dans laquelle ledit polypeptide est modifié par addition de groupements alkyle en C₁₋₁₂, addition de groupements hydroxyalkyle en C₁₋₁₂, addition de groupements acyle ou par cyclisation sous forme lactame.

6. Polypeptide isolé selon l'une quelconque des revendications 2 à 5, dans laquelle ledit polypeptide comprend une accumulation intracellulaire d'AMPc.

7. Polypeptide isolé selon la revendication 2, codé par la séquence d'acide nucléique de SEQ ID n° 14.

8. Séquence d'acide nucléique isolé codant pour le polypeptide selon l'une quelconque des revendications 2 à 5.

9. Utilisation d'un polypeptide selon l'une quelconque des revendications 2 à 5 dans la fabrication d'un médicament pour le traitement d'une hypercalcémie chez un mammifère ou d'une hypocalcémie chez un mammifère.

10. Utilisation d'un polypeptide selon l'une quelconque des revendications 2 à 5 dans la fabrication d'un réactif pour déterminer les vitesses de reformation osseuse, résorption osseuse et/ou remodelage osseux.

11. Utilisation d'ADN codant pour un polypeptide selon l'une quelconque des revendications 2 à 5, dans la fabrication d'un médicament pour utilisation dans le traitement d'une hypercalcémie chez un mammifère ou d'une hypocalcémie chez un mammifère, dans laquelle ledit polypeptide est exprimé in vitro.

12. Utilisation selon la revendication 9 ou la revendication 11, dans laquelle l'état à traiter est l'ostéoporose.

13. Utilisation selon la revendication 9, dans laquelle la dose dudit polypeptide est d'environ 0,01 µg/kg/jour à environ 1,0 µg/kg/jour.

14. Procédé in vitro d'augmentation de l'AMPc dans une cellule de mammifère ayant des récepteurs de la PTH-1 comprenant la mise en contact d'une cellule avec une quantité suffisante de polypeptide selon l'une quelconque des revendications 2 à 4.
